# EUROPEAN PATENT APPLICATION

(11) **EP 0 733 369 A1**
(43) Date of publication of application: **25.09.1996**
(21) Application number: 95200732.6
(22) Date of filing: 23.03.1995
(51) Int. Cl.: A61K 31/65, A61K 31/195, C07K 16/38, C12N 15/57, C12Q 1/37

(54) **Protease inhibitors, a DNA construct for the expression of a protease and a process for measuring proteases and/or protease inhibitors**

(71) Applicant: Stichting REGA V.Z.W., B-3000 Leuven (BE)
(72) Inventor: Opdenakker, Ghislain Marie Mathieu, B-9000 Gent (BE)
(74) Representative: Van Someren, Petronella F. H. M.

(57) **Abstract**

The invention relates to protease inhibitors, particularly metalloprotease inhibitors, for use in treating tissue destructive diseases such as multiple sclerosis, optic neuritis, arthritis and the like and for use in treating cancer; a DNA construct for the expression of the metalloprotease, gelatinase B; and also concerns a process for measuring proteases and/or protease inhibitors.

## Description

The present invention concerns protease inhibitors, particularly metalloprotease inhibitors, for use in treating tissue destructive diseases such as multiple sclerosis, optic neuritis, arthritis and the like and for use in treating cancer; a DNA construct for the expression of the metalloprotease, gelatinase B; and also concerns a process for measuring proteases and/or protease inhibitors.

Demyelination as observed in multiple sclerosis (MS) and experimental allergic encephalomyelitis (EAE) is an enzymatic process.

The enzymes, matrix metalloproteases, play a major role in extracellular matrix remodelling and are found in elevated amounts in the body fluids of patients with tissue destructive diseases such as arthritis (1,2), periodontitis (3), multiple sclerosis (4,5) and others (6).

Gelatinases are mammalian matrix metalloproteases which have been implicated as important mediators in physiological and pathological tissue remodelling including tumor invasion and metastasis (29; 38) and autoimmune diseases (26). The production and activity of these proteolytic enzymes are strictly controlled at several pre-and posttranscriptional levels. The latter include specific activation by a "cysteine switch" mechanism and inhibition by specific protease inhibitors, the tissue inhibitors of metalloproteases (TIMP) (6, 39). In humans, two different gelatinases, designated gelatinase A (72 kDa gelatinase) and gelatinase B (92 kDa gelatinase), have been described (40, 41, 7).

Gelatinase B (matrix metalloprotease-9, 90kDa) is a marker enzyme for acute and chronic inflammatory diseases including MS.

In contrast to the constitutive presence of gelatinase A (matrix metalloprotease-2, 70kDa), gelatinase B is found in the cerebrospinal fluid of patients with optic neuritis, MS or other inflammatory neurological diseases, but not in those of controls. In patients with multiple sclerosis the ratio of gelatinase B over gelatinase A is significantly correlated with the IgG index, the pathognomonic laboratory test for MS (9). In addition, gelatinase B has been shown to cleave myelin basic protein of human and other origin into fragments which correspond to encephalitogenic peptides in animal model systems and to the autoantigens against which specific T-cells are directed (10). Pathophysiologically, gelatinase B is produced in a regulated way by inflammatory cells. For instance the CXC chemokines IL-8 and GCP-2 stimulate neutrophils to produce gelatinase B and this might contribute to the histological picture in acute EAE (10,11). IL-1 and IL-2 induce the production of gelatinase B in monocytes and lymphocytes, respectively, and the former cell type can also be triggered by C-C chemokines to produce this enzyme. This might contribute to the enzymatic demyelination observed in MS (12).

Since there is evidence that enzymes, particularly proteases and more particularly metalloproteases are implicated with tissue destructive diseases and invasion and metastasis of cancer cells, inhibitors for these enzymes are sought after.

According to a first aspect, the present invention provides protease inhibitors, particularly metalloprotease inhibitors for use in treating tissue destructive diseases such as multiple sclerosis, optic neuritis, arthritis and the like.

According to a second aspect, the present invention provides protease inhibitors, particularly metalloprotease inhibitors for use in treating cancer.

The mechanism by which inhibitors affect matrix metalloprotease activity is not yet known. It has been suggested that the inhibition is linked to the ability to bind divalent ions such as Ca²⁺ and to interact with the catalytic Zn. Both mechanisms are essential for the activity of collagenase and gelatinase. Other evidence suggests that inhibitors can interact with cellular processes such as migration, degranulation and the synthesis of oxygen radicals.

D-penicillamine is currently used as an antirheumatic drug. Its mechanism of action was previously not known. The inventors have discovered that it inhibits gelatinase B and provide its mechanism of action.

Tetracyclines are widely therapeutically used as antimicrobial agents in all areas of medicine. However, tetracyclines also have important non-antimicrobial properties, which can influence the host response. Thus, tetracyclines have been shown to inhibit the activity of the matrix metalloproteases, collagenase and gelatinase both in vivo (19) and in vitro (20). The latter enzymes play a major role in extracellular matrix remodelling and are found in elevated amounts in the body fluids of patients with tissue destructive diseases such as arthritis (1,2), periodontitis (3), multiple sclerosis (4,5) and others (6).

The mechanism by which tetracyclines affect matrix metalloprotease activity is not yet known. It has been suggested that the inhibition is linked to the ability of tetracyclines to bind divalent ions such as Ca²+ and to interact with the catalytic Zn. Both mechanisms are essential for the activity of collagenase and gelatinase (21).

Other evidence suggests that tetracyclines can interact with cellular processes such as migration, degranulation and the synthesis of oxygen radicals (22). The matrix metalloprotease-inhibitory action of tetracyclines has become of such interest that tetracycline derivatives are being developed which have lost their antimicrobial properties altogether but retain their anti-matrix metalloprotease activities (23,24). These are of potential use in the aforementioned chronic inflammatory diseases.

The inventors have also defined macromolecular protease inhibitors.

Considering the metalloprotease inhibitors, these are preferably selected from the group consisting essentially of D-penicillamine, tetracyclines and derivatives therof and macromolecular compounds such as sera, antibodies, fragments and modified versions thereof which are directed against proteases.

These inhibitors yield optimum results in inhibiting proteases.

Matrix metalloproteases can also be inhibited by antibodies and derivatives thereof. The invention also provides polyclonal and monoclonal antibodies which are inhibitory.

Monoclonal antibodies were generated from mouse hybridomas. These were obtained by fusion of spleen cells from mice (, immunized with the human gelatinase B from neutrophils,) with a myeloma cell line. The resulting cultures were expanded and individual clones were selected by various methods. These included an ELISA method using as control reagents, the aforementioned purified gelatinase B and the polyclonal antisera from rabbits and mice. Another screening technique consisted in western blot analysis of purified gelatinase B and fragments thereof with the individual monoclonal antisera. With these two techniques several different monoclonals which bound gelatinase B were identified. To define the monoclonal antibodies which could inhibit the enzymatic activity of gelatinase B, hereafter called inhibitory monoclonal antibodies, we used the test system for gelatinase B inhibitors as claimed and an additional enzyme inhibition assay. The latter consisted in inhibition of gelatinase B activity as assayed by electrophoresis of cleaved gelatin fragments after digestion with a known amount of gelatinase B and increasing amounts of the inhibitory antibody to be tested.

Considering the proteases to be inhibited, these preferably belong to the group consisting essentially of gelatinases, plasminogen activators and processing proteases which are thought to be implicated in tissue destructive diseases and cancer.

Studies on the in vivo role of matrix metalloprotease and their specific inhibitors often have to rely on animal model systems for human diseases, for example mouse models for rheumatoid arthritis or cancer. In view of discrepancies in the published sequences of mouse gelatinase B (42, 43, 44, 45) and of the low production yields of gelatinase B glycoforms from cultured mouse cell lines (43), the inventors have expressed recombinant mouse gelatinase B in heterologous mammalian cells. In addition, the recombinant enzyme was purified to homogeneity by a single step procedure. The recombinant gelatinase B and derivatives, thus generated and generated by other expression systems in an unlimited amount is thus available for in vitro and in vivo testing and is defined by this primary sequence.

The amino acid sequence of the mouse gelatinase B was described in the publication "Mouse gelatinase B: cDNA cloning, regulation of expression and glycosylation in WEHI-3 macrophages and gene organisation", S. Masure, G. Nys, P. Fiten, J. Van Damme, G. Opdenakker (1993) (43) and is as follows:

gDNA TTAGCCAGAAGCTGCGGTCCTCACCATGAGTCCCTGGCAGCCCCTGCTCCTGGCTCTCCTGGCTTTCGGCTGCAGCTCTGCTGCCCCTTACCAGCGCCAG 100
AAgDNA M S P W Q P L L L A L L A F G C S S A A P Y Q R Q 25

gDNA CCGACTTTTGTGGTCTTCCCCAAAGACCTGAAAACCTCCAACCTCACGGACACCCAGCTGGCAGAGGCATACTTGTACCGCTATGGTTACACCCGGGCCG 200
AAgDNA P T F V V F P K D L K T S N L T D T Q L A E A Y L Y R Y G Y T R A 58

gDNA CCCAGATGATGGGAGAGAAGCAGTCTCTACGGCCGGCTTTGCTGATGCTTCAGAAGCAGCTCTCCCTGCCCCAGACTGGTGAGCTGGACAGCCAGACACT 300
AAgDNA A Q M M G E K Q S L R P A L L M L Q K Q L S L P Q T G E L D S Q T L 92

gDNA AAAGGCCATTCGAACACCACGCTGTGGTGTCCCAGACGTGGGTCGATTCCAAACCTTCAAAGGCCTCAAGTGGGACCATCATAACATCACATACTGGATC 400
AAgDNA K A I R T P R C G V P D V G R F Q T F K G L K W D H H N I T Y W I 125

cDNA
gDNA CAAAACTACTCTGAAGACTT GCCGCGAGACATGATCGATGACGCCTTCGCGCGCGCCTTCGCGGTGTGGGGCGAGGTGGCACCCCTCACCTTCACCCGCG 500
AAcDNA
AAgDNA Q N Y S E D L P R D M I D D A F A R A F A V W G E V A P L T F T R 158

cDNA
gDNA TGTACGGACCCGAAGCGGACATTGTCATCCAGTTTGGTGTCGCGGAGCACGGAGACGGGTATCCCTTCGACGGCAAGGACGGCCTTCTGGCACACGCCTT 600
AAcDNA
AAgDNA V Y G P E A D I V I Q F G V A E H G D G Y P F D G K D G L L A H A F 192

cDNA
gDNA TCCCCCTGGCGCCGGCGTTCAGGGAGATGCCCATTTCGACGACGACGAGTTGTGGTCGCTGGGCAAAGGCGTCGTGATCCCCACTTACTATGGAAACTCA 700
AAcDNA
AAgDNA P P G A G V Q G D A H F D D D E L W S L G K G V V I P T Y Y G N S 225

cDNA
gDNA AATGGTGCCCCATGTCACTTTCCCTTCACCTTCGAGGGACGCTCCTATTCGGCCTGCACCACAGACGGCCGCAACGACGGCACGCCTTGGTGTAGCACAA 800
AAcDNA
AAgDNA N G A P C H F P F T F E G R S Y S A C T T D G R N D G T P W C S T 258

cDNA
gDNA CAGCTGACTACGATAAGGACGGCAAATTTGGTTTCTGCCCTAGTGAGAGACTCTACACGGAGCACGGCAACGGAGAAGGCAAACCCTGTGTGTTCCCGTT 900
AAcDNA
AAgDNA T A D Y D K D G K F G F C P S E R L Y T E H G N G E G K P C V F P F 292

cDNA
gDNA CATCTTTGAGGGCCGCTCCTACTCTGCCTGCACCACTAAAGGCCGCTCGGATGGTTACCGCTGGTGCGCCACCACAGCCAACTATGACCAGGATAAACTG 1000
AAcDNA
AAgDNA I F E G R S Y S A C T T K G R S D G Y R W C A T T A N Y D Q D K L 325

cDNA
gDNA TATGGCTTCTGCCCTACCCGAGTGGACGCGACCGTAGTTGGGGGCAACTCGGCAGGAGAGCTGTGCGTCTTCCCCTTCGTCTTCCTGGGCAAGCAGTACT 1100
AAcDNA
AAgDNA Y G F C P T R V D A T V V G G N S A G E L C V F P F V F L G K Q Y 358

cDNA
gDNA CTTCCTGTACCAGCGACGGCCGCAGGGATGGGCGCCTCTGGTGTGCGACCACATCGAACTTCGACACTGACAAGAAGTGGGGTTTCTGTCCAGACCAAGG 1200
AAcDNA
AAgDNA S S C T S D G R R D G R L W C A T T S N F D T D K K W G F C P D Q G 392

cDNA
gDNA GTACAGCCTGTTCCTGGTGGCAGCGCACGAGTTCGGCCATGCACTGGGCTTAGATCATTCCAGCGTGCCGGAAGCGCTCATGTACCCGCTGTATAGCTAC 1300
AAcDNA
AAgDNA Y S L F L V A A H E F G H A L G L D H S S V P E A L M Y P L Y S Y 425

cDNA
gDNA CTCGAGGGCTTCCCTCTGAATAAAGACGACATAGACGGCATCCAGTATCTGTATGGTCGTGGCTCTAAGCCTGACCCAAGGCCTCCAGCCACCACCACAA 1400
AAcDNA
AAgDNA L E G F P L N K D D I D G I Q Y L Y G R G S K P D P R P P A T T T 458

cDNA
gDNA CTGAACCACAGCCGACAGCACCTCCCACTATGTGTCCCACTATACCTCCCACGGCCTATCCCACAGTGGGCCCCACGGTTGGCCCTACAGGCGCCCCCTC 1500
AAcDNA
AAgDNA T E P Q P T A P P T M C P T I P P T A Y P T V G P T V G P T G A P S 492

cDNA
gDNA ACCTGGCCCCACAAGCAGCCCGTCACCTGGCCCTACAGGCGCCCCCTCACCTGGCCCTACAGC[G/C]CCCCTACTGCGGGCTCTTCTGAGGCCTCTACAGAG 1600
AAcDNA
AAgDNA P G P T S S P S P G P T G A P S P G P T A [A/P] P T A G S S E A S T E 525

cDNA
gDNA TCTTTGAGTCCGGCAGACAATCCTTGCAATGTGGATGTTTTTGATGCTATTGCTGAGATCCAGGGCGCTCTGCATTTCTTCAAGGACGGTTGGTACTGGA 1700
AAcDNA
AAgDNA S L S P A D N P C N V D V F D A I A E I Q G A L H F F K D G W Y W 558

cDNA
gDNA AGTTCCTGAATCATAGAGGAAGCCCATTACAGGGCCCCTTCCTTACTGCCCGCACGTGGCCAGCCCTGCCTGCAACGCTGGACTCCGCCTTTGAGGATCC 1800
AAcDNA
AAgDNA K F L N H R G S P L Q G P F L T A R T W P A L P A T L D S A F E D P 592

cDNA
gDNA GCAGACCAAGAGGGTTTTCTTCTTCTCTGGACGTCAAATGTGGGTGTACACAGGCAAGACCGTGCTGGGCCCCAGGAGTCTGGATAAGTTGGGTCTAGGC 1900
AAcDNA
AAgDNA Q T K R V F F F S G R Q M W V Y T G K T V L G P R S L D K L G L G 625

cDNA
gDNA CCAGAGGTAACCCACGTCAGCGGGCTTCTCCCGCGTCGTC[C/T]GGGAAGGCTCTGCTGTTCAGCAAGGGGCGTGTCTGGAGATTCGA[T/C]TTGAAGTCTCAGA 2000
AAcDNA
AAgDNA P E V T H V S G L L P R R [P/L] G K A L L F S K G R V W R F [D] L K S Q 658

cDNA
gDNA AGGTGGATCCCCAGAGCGTCATTCGCGTGGATAAGGAGTTCTCTGGTGTGCCCTGGAACTCACACGACATCTTCCAGTACCAAGACAAAGCCTATTTCTG 2100
AAcDNA
AAgDNA K V D P Q S V I R V D K E F S G V P W N S H D I F Q Y Q D K A Y F C 692

cDNA
gDNA CCATGGCAAATTCTTCTGGCGTGTGAGTTTCCAAAATGAGGTGAACAAGGTGGAC[C/A]TGAGGTGAACCAGGTGGACGACGTGGGCTACGTGACCTACGAC 2200
AAcDNA
AAgDNA H G K F F W R V S F Q N E V N K V D [P/H] E V N Q V D D V G Y V T Y D 725

cDNA
gDNA CTCCTGCAGTGCCCTTGAACTAGGGCTCCTTCTTTGCTTCAACCGTGCAGTGCAAGTCTCTAGAGACCACCACCACCACCACCACACACAAACCCCCATCC 2300
AAcDNA
AAgDNA L L Q C P

cDNA
gDNA GAGGGAAAGGTGCTAGCTGGCCAGGTACAGACTGGTGATCTCTTCTAGAGACTGGGAAGGAGTGGAGGCAGGCAGGGCTCTCTCTGCCCACCGTCCTTTC 2400

cDNA TTGTTGGACTGTTTCTAATAAACACGGATCCCCAACCTTAAAAAAAAAAAAAAAAAAAAAAAA...
gDNA TTCCAGCTACTTTAGTCAATCAGCT...

Figure A

According to another aspect of the present invention there is provided a construct comprising a transcription initiation region, at least a part of the DNA-sequence according to figure A and a termination region aligned in the proper reading frame.

In contrast to collagenases, for which quantitative chromogenic substrate release and substrate conversion assays exist, gelatinases are commonly quantified by zymographic procedures (7,8). In the zymographic assay, gelatinases are dissociated from their natural inhibitors and are activated. This impairs the quantification of the net activities of the enzyme and enzyme inhibitor in biological and other samples. Previously, quantitative zymography was used to measure gelatinases in body and tissue culture fluids (1,4,5,7,8). Natural inhibitors of metalloproteases can not be measured by direct zymography. Indirect zymography-based inhibitor assays can only be used for small diffusable molecules and require a substantial amount of inhibitor. Finally, reverse zymography as an assay for enzyme inhibitors is labour-intensive and thus not practical.

Furthermore the inhibitory activity of antisera and monoclonal antibodies can not be tested using zymography because the antibodies need to diffuse into the substrate gel. The same holds true for other macromolecular inhibitors of gelatinases, for instance tissue-inhibitors of metalloproteases.

There is thus a demand for a quantitative measurement for proteases and protease inhibitors.

According to yet a further aspect of the present invention there is provided a process for measuring proteases and/or protease inhibitors comprising the following steps:
a) digestion of a predetermined substrate by the protease followed by
b) quantification of the residual non-digested substrate.

This in vitro assay is based on degradation of biotinylated gelatin, one of the substrates for gelatinases. The assay is useful for the discovery of gelatinase inhibitors, both macromolecular ones and small inhibitors.

By means of this assay the inventors were able to define the macromolecular protease inhibitors referred to above.

The invention is further illustrated by the following examples which should not be considered as limiting the scope of the present invention.

### EXAMPLES

### EXAMPLE 1

### Assay system and derived assay systems to measure gelatinases and in particular gelatinase inhibitors

### 1.1. Introduction

A quantitative non-isotopic solution assay for gelatinases and inhibitors was developed using biotinylated gelatin as the enzyme substrate. In this assay residual substrate is sandwiched between avidin-coated plates and streptavidin-peroxidase and is quantified by the peroxidase reaction. This assay was useful to measure gelatinase activities in crude and purified samples (current detection limit 3 ng gelatinase B) and to define the activities of gelatinase inhibitors. The assay was demonstrated to be useful to discover gelatinase B inhibitory molecules.

### 1.2. Detailed description of the assay

### Reagents

The gelatin, avidin and biotin hydroxysuccinimide ester were purchased from Sigma Chemical Company, St. Louis, USA. The streptavidin-peroxidase complex was obtained from Jackson ImmunoResearch Laboratories, Pennsylvania, USA. The peroxidase color reagent Azinobenzthiazolium salt (ABTS) was purchased from Boehringer Mannheim, Germany. Alkaline-phosphatase-conjugated anti-rabbit and anti-mouse antisera were purchased from DAKO/PROSAN, Glostrup, Denmark.

### Gelatinase microplate assay

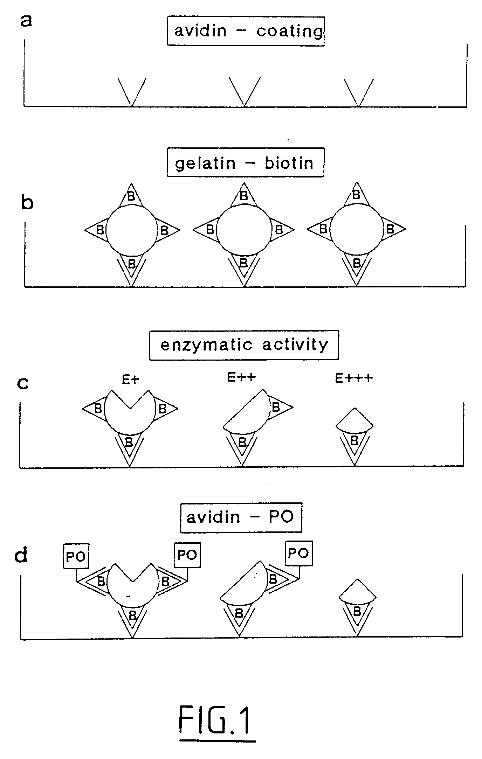

The measurement of gelatinase activity was based on the detection of non-degraded biotinylated gelatin with a streptavidin-peroxidase conjugate. This test is based on the biotin-avidin method described by Sorbi et al. (25) but modified to a one plate assay (schematically represented in Fig. 1.). Gelatin (1mg/ml) was dissolved in 0.1 M sodium bicarbonate pH 8.3 by heating to 40°C. Biotin-N-hydroxysuccinimide ester was dissolved in dimethylsulfoxide (Me₂SO) and was added to the gelatin in a 15-fold molar excess. The mixture was allowed to react overnight at 4°C and the biotinylated gelatin was then dialyzed against phosphate buffered saline (PBS). Neutrophil granulocyte (Neu) medium was collected from human neutrophils after stimulation with lipopolysaccharide (1µg/ml) overnight at 37°C in Minimal Essential Medium. Cells were removed by 10 min centrifugation at 1000 g. Purified gelatinase was obtained by one-step affinity chromatography of neutrophil medium on a gelatin-sepharose column as described (8).

Modified flat bottom 96-well ELISA plates were coated overnight with 0.5 µg/ml avidin in distilled water. The plates were then blocked with 0.3% bovine serum albumin (BSA, Sigma) in PBS for 1 hour at 37°C. Biotinylated gelatin was diluted to 50 µg/ml and 100µl was added to the avidin-coated wells. After incubation for one hour at 20°C, the plate was rinsed with PBS. Medium containing the secreted gelatinases from human neutrophils (dilution range 1/3-1/3¹¹) and the inhibitors were diluted in incubation buffer (50 mM Tris-HCl pH 7.5, 10 mM CaCl₂, 0.02% (W/V) NaN₃, 1% (V/V) Triton X-100) and 100 µl of each were mixed. 190 µl of the mixture was added to the gelatin-coated plates and allowed to digest the gelatin overnight at 37°C. The plates were then washed with PBS and each well was incubated with 100 µl peroxidase-conjugated-streptavidin at a 1:2000 dilution for 1 hour at 37°C. After rinsing the plate, the ABTS substrate was added (0.5 mg/ml solution containing 0.1 M citric acid, 0.2 M Na₂HPO₄, pH 4.3, 0.07 µl/ml 30% H₂O₂) and the optical density was read at 405 nm after 10 min. incubation at room temperature.
Additional information on published test systems used here for comparison.

### Zymography

Quantitative zymography was performed as described (7,8). Briefly, 40 µl of loading buffer was added to 40 µl of each sample and applied on 7.5% polyacrylamide gels which had been co-polymerised with 0.1% (w/v) gelatin. Stacking gels contained 5% polyacrylamide. The gels were run at 4°C for 16h at 90V. The gels were then incubated in washing buffer (50 mM Tris-HCl pH 7.5, 10mM CaCl₂, O.O2% (W/V) NaN₃, 2.5% (v/v) Triton X-100) at room temperature, 2x20 min to remove sodium dodecyl sulfate and overnight in developing buffer (50 mM Tris-HCl pH 7.5, 10mM CaCl₂, O.O2% (w/v) NaN₃, 1% (v/v) Triton X-100) at 37°C. The gels were then stained in Coomassie brilliant blue R-250 and destained in methanol/acetic acid. The sites of gelatinase activity appeared as unstained bands on a blue background and were quantified by computerised image analysis through scanning densitometry with a PDI scanner (Pharmacia, Belgium) and the PDI software package. For inhibition experiments, the inhibitors were dissolved in the developing buffer at the appropriate concentration.

### Western blotting

Standard neutrophil gelatinase (1µl) was seperated on a 15% SDS/PAGE gel and transferred to a polyvinyldifluoridon (PVDF) membrane. The membranes were blocked in 3% bovine albumin serum in Tris-buffered saline (TBS) for one hour and then incubated overnight in a 1/5000 dilution of the primary antisera in TBS. After washing, the membranes were incubated with an alkaline-phosphatase-conjugated anti-rabbit or anti-mouse antibody (1/2000 dilution), depending on the origin of the primary antibody, and stained with nitroblue tetrazolium and bromochloroindolylsulphate until bands appeared.

### Gelatinase inhibitor microplate assay

A plate assay for the quantification of gelatinases and their inhibitors was established (Fig. 1). Gelatin is biotinylated and bound to avidin-coated microtiter plates. By addition of gelatinases the number of free biotin groups decreases and proportionally less streptavidin-peroxidase is bound to the residual substrate. Bound streptavidin-peroxidase activity is measured spectrophotometrically by reaction of peroxide with ABTS. Inhibition of gelatinase is obtained by the addition of inhibitor to a known amount of gelatinase prior to application to the plates. The peroxidase activity increases proportionally with the inhibitory activity.

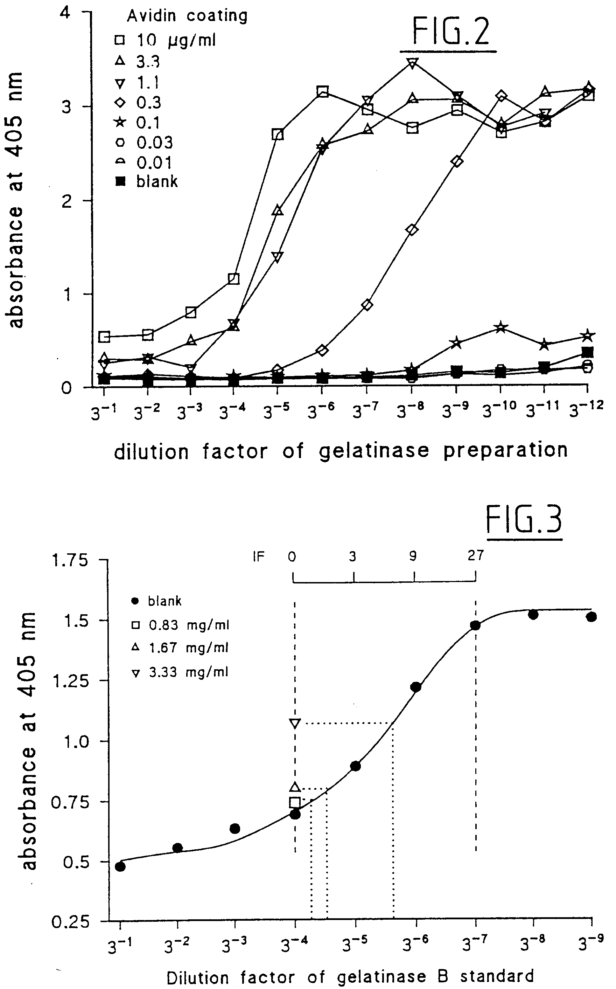

Plates were first coated with 10µg/ml avidin and 50µg/ml biotinylated gelatin (theoretically saturating the avidin binding sites). As the sensitivity of the assay is dependent on the detection of the minimal amount of degraded biotinylated gelatin, we investigated wether decreasing the amount of biotin-gelatin (50 µg/ml-23 ng/ml) bound to the avidin-coated (10µg/ml) plate increased the sensitivity of the assay. However, we found that non-saturation of the avidin did not enhance the sensitivity significantly. Thus it was verified whether a decrease in the avidin coating could make the assay more sensitive (Fig. 2). The optimal avidin concentration for the detection of decreasing gelatinase B activity was determined at 0.3-1.1 µg/ml (Fig. 2). In all further examples plates were coated with 0.5 µg/ml avidin and 50 µg/ml gelatin-biotin.

### Titration of gelatinase B in the microtiter plate assay

On a theoretical basis the developed test is useful for both enzyme and enzyme-inhibitor assays.

Previously, the inventors have shown that neutrophils do not produce the natural tissue inhibitor of metalloprotease (TIMP-1), or gelatinase A (8). In the examples of the inventors a standard preparation, containing purified homogenous gelatinase B from human neutrophils yielded a titration curve parallel with that of crude preparations of neutrophil gelatinase B in the plate assay. For all further inhibition experiments, the crude preparation of neutrophil gelatinase B was used.

The change in optical density with respect to the change in enzyme concentration is sigmoidal (Fig. 3). The titration curves of gelatinase inhibition were always related to a blanc curve and this for enzyme concentrations within the slope of the curve (vertical dotted lines Fig. 3). The decrease in enzyme activity to which the inhibitor-incubated sample corresponded was defined as the inhibition factor (IF).

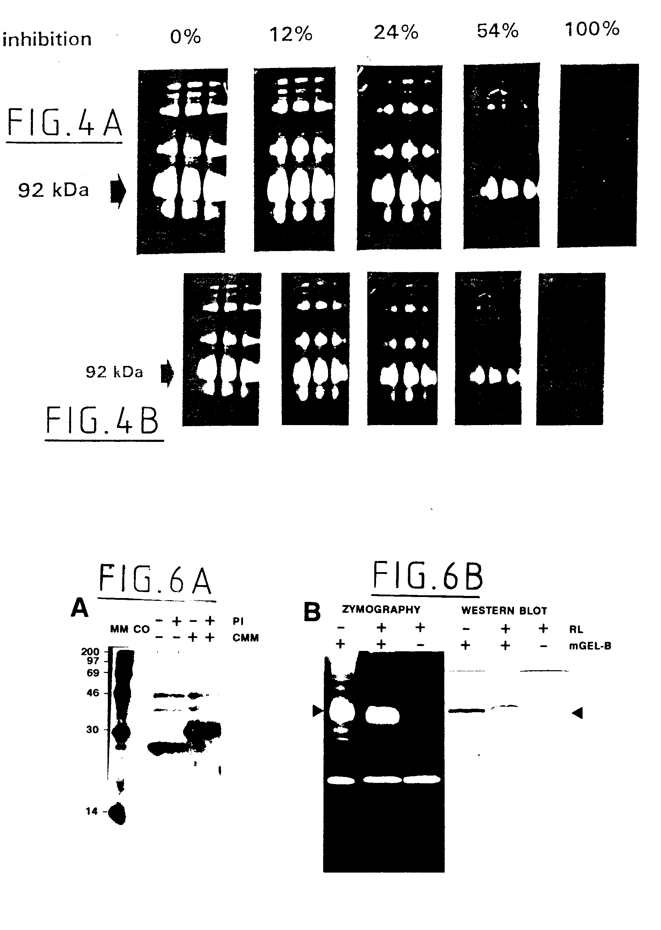

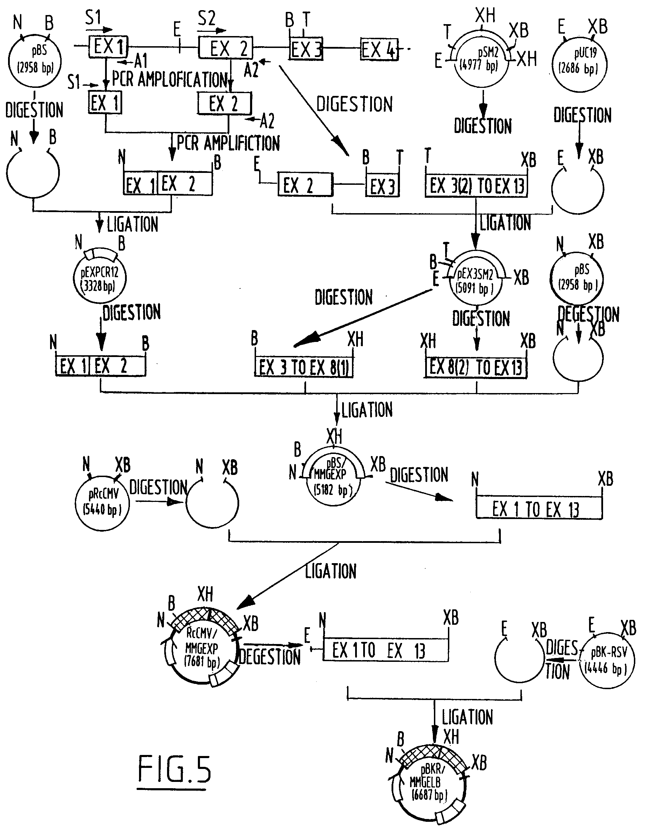

With the classical zymography technique it was found that minocyclin inhibits gelatinase B in a dose-dependent way (Fig. 4). Therefore, this inhibitor was tested for its inhibitory acitivity in the gelatinase microtiter plate assay and found to be active as an inhibitor.

### 1.3. Summary

The invented and described assay is based on the digestion of biotinylated gelatin by gelatinases and quantification of residual biotinylated gelatin by peroxidase-streptavidin. With the use of laboratory standard preparations this microtiter plate assay can be employed to quantify gelatinases in crude supernatants and to test for inhibitors of gelatinases.

In contrast to the zymography inhibitor assays, the microtiter plate assay is less labour-intensive and can be performed with at least 1000-fold less inhibitor. To exemplify its usefulness and to determine the structure-function effects we studied the inhibitory effect of a tetracycline. Often biological fluids are complex mixtures of both enzymes and enzyme inhibitors. The natural inhibitors of matrix metalloproteases are tissue inhibitors and these are dissociated from the enzyme in the zymography assays (7). In other words, whith the quantitative zymography technique free SDS-activated enzyme activity is measured without inhibitors. Because the microtiter plate assay is essentially a solution assay on a solid phase substrate, with the latter assay the net gelatinolytic activity is measured of both enzymes and inhibitors. Other techniques were tried as a means to quantify gelatinolytic activity in solution. These methods consist of a) an in vitro digestion of commercially available or iodinated gelatin, b) SDS-PAGE of the substrate and reaction products, c) staining analysis or autoradiography and d) scanning densitometry. The following drawbacks hampered their practical use: major batch differences in the substrate, too many bands to be analysed in the substrate and reaction products and the need for a specific laboratory environment for the isotopic method. The microtiter plate assay is a fairly simple, non-isotopic gelatinase assay which allows measurement of net gelatinase activity in biological samples as well as the titration of gelatinase inhibitory substances. In addition, this assay can be modified easily as to be adapted to semi-automated or fully automated robotics.

### 1.4. Legends to the figures

Figure 1. Flow chart of the gelatinase microtiter plate assay. Individual wells (illustration) of a microtiter plate are coated with avidin (a) to which biotinylated gelatin substrate is bound (b). Enzymatic activity (E) of gelatinases liberate gelatin fragments and decrease proportionally the number of residual biotin (c). The latter is spectrophotometrically quantified by binding of streptavidin-peroxidase and development of peroxidase activity (PO) using peroxide and azinobenzthiazolium salt as a color reagent.

Figure 2. Effect of avidin coating on the sensitivity of the gelatinase microplate assay. Plates were coated with decreasing amounts (10µg-10ng) or without (blank) avidin and after blocking incubated with 50 µg/ml gelatin-biotin. The ability of the assay to detect the effect of decreasing amounts of gelatinase B standard preparation on the degradation of gelatin is measured by streptavidin-peroxidase binding to residual biotinylated gelatin and development of peroxidase activity. The standard preparation contained an equivalent of 60 µg/ml gelatinase activity.

Figure 3. Determination of the inhibition factor. A standard preparation of gelatinase B from human neutrophils was spectrophotometrically titrated in the microtiter plate assay by digesting biotinylated gelatin. Addition of inhibitor (3.33 mg/ml to 0.09 mg/ml shifted the titration curves in a parallel way and enabled to determine the inhibitory factor (IF).

Figure 4. Inhibition of gelatinase B by minocycline on zymography. A standard preparation of gelatinase B from neutrophils was repetitiously loaded in the slots of a zymography gelatin substrate gel. After electrophoretic separation the gel was sliced every three lanes and the slices were incubated in test tubes in developing buffer without and with increasing amounts of gelatinase B inhibitor. The enzyme activity was developed by staining of the substrate with Coomassie blue and quantification of the substrate digestion by scanning densitometry. Percent inhibition is related to a control to which no minocycline was added. The 92 kDa neutrophil gelatinase is indicated with an arrowhead.

### EXAMPLE 2

### Recombinant mouse gelatinase B and derivatives for gelatinase and gelatinase inhibitor assays, for in vitro and in vivo applications.

### 2.1. Introduction

Cloned DNA of Mouse gelatinase B, a matrix metalloprotease involved in tissue remodelling, expressed in Chinese hamster ovary (CHO) cells and translated from its mRNA in reticulocyte lysates.

By PCR-mediated exon amplification, hybrid genomic DNA/cDNA constructs were generated under the control of two constitutive viral promoters. In vitro transcription generated an intact mRNA species yielding by reticulocyte lysate translation three gelatinase B fragments. The aminoterminal fragment was shown to be glycosylated. Transfection of the hybrid gelatinase B DNA in CHO cells yielded intact recombinant enzyme in addition to processed gelatinase B fragments. Recombinant 110 kDa gelatinase B was purified by a single step affinity chromatography procedure and shown to comigrate with the natural gelatinase B from WEHI-3 macrophages, to immunoprecipitate with a specific antiserum and to be enzymatically active.

Abbreviations. CHO, Chinese hamster ovary; DMEM, Dulbecco's modified Eagle medium; MMP, matrix metalloprotease; PMSF, phenylmethylsulfonyl fluoride; PVDF, polyvinylidene difluoride; TIMP, tissue inhibitor of metalloproteases. Enzymes. Gelatinase A (72 kDa gelatinase, matrix metalloprotease-2; EC 3.4.24.24); Gelatinase B (92 kDa gelatinase, matrix metalloprotese-9; EC 3.4.24.35).

### 2.2. Experimental procedures

### Construction of mouse gelatinase B expression plasmids

In order to make a DNA construct for the expression of the mouse gelatinase B in eukaryotic cells, a genomic DNA/cDNA hybrid was synthesized. This construction was necessary since only partial cDNA clones coding for the gelatinase B from mouse WEHI-3 macrophages could be isolated (43). A cDNA fragment from a partial gelatinase B cDNA clone (pSM2; coding for part of exon 3 and for exons 4 to 13) was coupled to the genomic DNA coding for exons 1 and 2 and for the missing part of exon 3 (Figure 5).

First, a partial construct was made (pEX3SM2) by ligating 1) a 694 bp EcoRI-TthIII1 fragment from a genomic cosmid DNA subclone (containing the DNA from part of intron 1-2, exon 2, intron 2-3 and part of exon 3); 2) a 1738 bp TthIII1-XbaI fragment from the cDNA clone pSM2 (other part of exon 3 plus exons 4 to 13 + 41 bp of 3'-untranslated DNA) and 3) the pUC19 plasmid digested with EcoRI and XbaI.

Exon 1 was coupled to exon 2 by PCR amplification using custom-made "sticky feet primers". First, exon 1 was amplified using a sense primer for exon 1 (SPEX1 = 5'-CATGCGGCCGCCATGAGTCCCTGGCAGC-3') and an antisense primer for exon 1 (APEX1 = 5'-ACAAGTATGC/CTCTGCCAGCTGGGTGTC-3'). The 5'-end of SPEX1 contains a NotI restriction site (underlined) just upstream of the ATG initiation codon. At the 5'-end of APEX1, there is a 10 bp extension (in italics) complementary to the first 5' 10 base pairs of exon 2. Exon 2 was amplified using two other primers, SPEX2 (= 5'-GCTGGCAGAG/GCATACTTGTACCGCTATGG-3') and APEX2 (= 5'-TTGGATCCAGTATGTGATGTTATGATGG-3'). The first 10 base pairs of SPEX2 (in italics) are complementary to the 3'-end of exon 1. APEX2 contains at its end a BamHI restriction site (underlined) which is located exactly at the boundary between exon 2 and exon 3. PCR amplifications were performed using the following conditions: 1 min at 95°C, 50 s at 55°C and 40 s at 72°C (30 cycles) in the presence of 10 ng of template cosmid DNA. After electrophoresis of the PCR reaction products in 1.5% agarose, bands of the expected exon size (163 bp for exon 1 and 247 bp for exon 2, respectively) were eluted from the gel. Exon 1 and 2 were joined by performing 7 PCR cycles (1 min at 95°C; 45 s at 55°C; 55 s at 72°C) in the absence of primers, followed by another 25 cycles in the presence of 25 pmol of the outward primers SPEX1 and APEX2.

The resulting 390 bp PCR product was digested with NotI and BamHI and subcloned in the plasmid pBluescript. From the resulting plasmid pEXPCR12, the fragment could be excised with NotI and BamHI. The sequence of the 378 bp insert was determined and corresponded exactly to the genomic sequence of exons 1 and 2.

In a subsequent step, four DNA fragments were joined in a single ligation reaction: 1) the 378 bp NotI-BamHI fragment from plasmid pEXPCR12 (exons 1 and 2); 2) a 905 bp BamHI-XhoI fragment isolated from plasmid pEX3SM2 (exon 3 to first part of exon 8); 3) a 958 bp XhoI-XbaI fragment also isolated from plasmid pEX3SM2 (second part of exon 8 to exon 13 and 41 bp of 3'-untranslated DNA); and 4) the pBluescript plasmid digested with NotI and XbaI. The resulting construct (pBS/MMGEXP) was verified by partial sequence analysis and by restriction analysis. The 2241 bp insert contained 503 bp derived from genomic DNA (exon 1, 2 and part of 3) and 1738 bp derived from cDNA (part of exon 3, exons 4 to 13 and 41 bp of 3'-untranslated DNA). The complete mouse gelatinase B coding sequence was then cloned into appropriate eukaryotic expression vectors: in pRcCMV (Invitrogen Corporation) under the control of the viral CMV-promoter (RcCMV/MMGEXP) and in pBK-RSV (Stratagene) under the control of the viral RSV-promoter (pBKR/MMGELB).

### In vitro transcription and translation of the hybrid construct

For the in vitro production of recombinant mouse gelatinase B, the expression plasmid pBKR/MMGELB was linearized with the restriction endonuclease SspI and treated with protease K. mRNA was transcribed from 4 µg template DNA with 45 U T3 RNA polymerase according to the instructions of the manufacturer (Promega Corporation). After DNAse treatment, 2 µg of the transcription product was translated in rabbit reticulocyte lysate in the presence of [³⁵S]methionine and with or without addition of canine microsomal membranes. Antipain (final concentration 100 µM) and phenylmethylsulfonyl fluoride (PMSF; final concentration 0.8 µM) were included in certain translation experiments to inhibit proteases possibly present in the reticulocyte lysate preparations. Aliquots of the in vitro translation mixes were run on 12.5% acrylamide gels. Gels were vacuum dried and autoradiographed after amplification with Amplify (Amersham).

### Expression of mouse gelatinase B in Chinese hamster ovary (CHO) cells

### Expression of mouse gelatinase B in Chinese hamster ovary (CHO) cells

Plasmid DNAs from the expression constructs (RcCMV/MMGEXP or pBKR/MMGELB) were linearized with BglII or SspI, respectively, extracted twice with phenol and precipitated with ethanol. 40 µg of this linearized DNA was then used to transfect CHO cells by electroporation (46). Control electroporation experiments were performed using linearized plasmid DNA from a pRcCMV or pBK-RSV vector-derivative. After 2 days of culture in DMEM containing 10% fetal bovine serum, antibiotic G-418 (geneticin, BRL Life Technologies) was added at a final concentration of 0.5 or 1 mg/ml. Cells were incubated further in the presence of G-418, the medium was changed every 2 to 3 days and cell cultures were inspected for the formation of G-418-resistant colonies. Single or pooled colonies were subcloned in DMEM containing 500 µg/ml of G-418, grown to confluency in the absence of serum and their supernatants tested for gelatinase activity by zymography.

### Detection and purification of recombinant gelatinase B activity

The gelatinase activity in cell culture supernatants was detected by gelatin-substrate zymography as described. As previously shown this assay is specific and yields information about the gelatinase molecular weight (7). Recombinant murine gelatinase B enzyme was purified on mini-columns of gelatin-Sepharose as described (8), with minor modifications.

### Western blotting

After electrophoresis in 15% acrylamide gels, proteins were transferred to PVDF membranes and immunostained using a rabbit polyclonal antibody raised against human gelatinase B (8). Briefly, membranes were incubated for 1 h in 3% bovine serum albumin in Tris-buffered saline and then in a 1/2000 dilution of the primary dilution) and then stained with nitroblue tetrazolium and bromochloroindolylsulphate until bands appeared.

### In vitro transcription and translation of murine gelatinase B

To assess the functionality of the hybrid genomic DNA/cDNA expression construct, plasmid DNA from pBKR/MMGELB was transcribed in vitro using T3 RNA polymerase and the mRNA translated in rabbit reticulocyte lysate. One single mRNA species, hybridisable to mouse gelatinase B cDNA, was found by Northern blot analysis. Three [³⁵S]-labelled protein fragments with relative molecular masses of 45, 38 and 26 kDa respectively were visible on SDS/PAGE (Figure 6A). Addition of canine microsomal membranes to the in vitro translation mix also resulted in the production of three fragments, but the lower band migrated at a Mr of 30 instead of 26 kDa. The presence of serine and cysteine protease inhibitors during translation did not prevent the appearance of the three discrete protein bands. To assess the direct effect of reticulocyte lysate treatment on murine gelatinase B and to account for the differences in the proteins produced in reticulocyte lysates and in transfected CHO cells (see below), purified recombinant enzyme from pBKR/MMGELB-transfected cells was incubated with rabbit reticulocyte lysate and then analysed by zymography and Western blotting (Figure 6B). Reticulocyte lysate treatment did not result in processing of the 110 kDa band to lower Mr fragments.

### Expression of murine gelatinase B in CHO cells

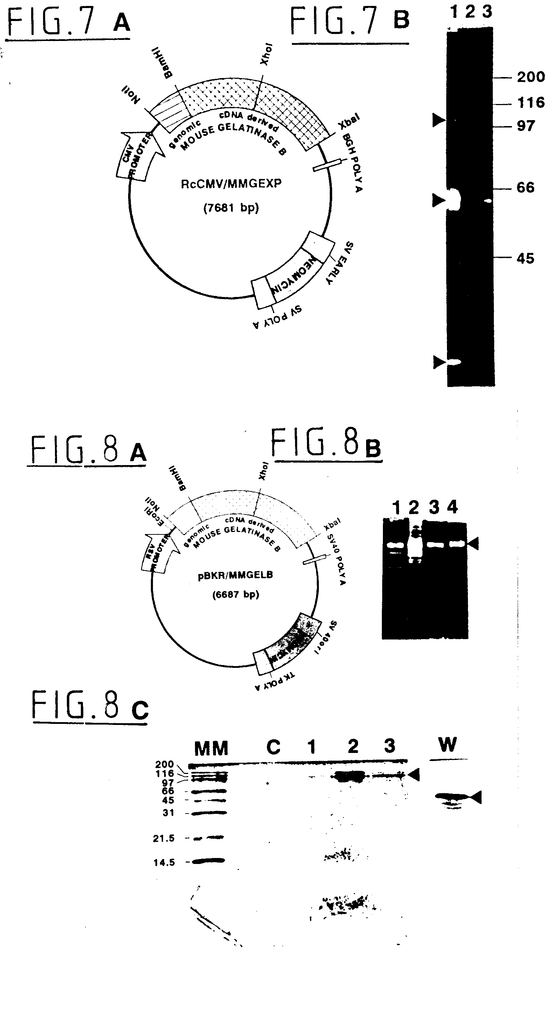

CHO cells were transfected with the RcCMV/MMGEXP construct (Figure 7A) by electroporation. After G-418 selection, pools of clones were grown to confluency and their supernatants tested for gelatinase activity by zymography (Figure 7B). Supernatants from untreated CHO cells (lane 3) and control-transfected CHO cells (lane 2) contained endogenous hamster gelatinases A and B migrating at 65 kDa and 95 kDa, respectively, on zymography. In the RcCMV/MMGEXP-transfected cells (lane 1), additional gelatinase bands migrating at 110 kDa, 66 kDa and less than 45 kDa were present and represented intact recombinant murine gelatinase B (110 kDa) and presumably processed murine gelatinase B fragments.

After transfection of CHO cells with pBKR/MMGELB plasmid DNA (Figure 8A) and G-418 selection, individual clones or pools of clones were grown to confluency, then incubated for 24 h in serum-free medium and the supernatants tested for additional gelatinase activity compared to non-transfected and control-transfected CHO cells. Screening of more than 60 individual clones resistant to antibiotic G-418 from 4 different transfection experiments did not yield clones producing silver-stainable proteins or gelatinolytic activity on zymography different from those present in control supernatants. One pool of G-418 resistant clones, however, produced an intense protein band on silver-stained SDS/PAGE not present in the controls (Figure 8C, lane 2). This protein could also be detected as an additional gelatinolytic activity with Mr around 110 kDa on zymography (Figure 8B, lane 1). Additional experiments confirmed this protein to represent recombinant mouse gelatinase B: the activity could be purified on gelatin-Sepharose and the protein reacted with a polyclonal gelatinase B antiserum on Western blot. Moreover, the activity comigrated with natural gelatinase B from mouse macrophages (WEHI-3 cell line) (Figure 8B and C).

### 2.3. Summary

A hybrid genomic DNA/cDNA plasmid construct containing the complete mouse gelatinase B coding sequence was used to produce recombinant mouse gelatinase B enzyme. Transfection of CHO cells produced high amounts of a 110 kDa protein with gelatin-degrading capacity. The three reticulocyte lysate translation products represent fragmented mouse gelatinase B, since their respective Mr add up to approximately 105 kDa without processing and approximately 110 kDa after posttranslational processing by canine microsomal membranes. In accordance with this, treatment of purified recombinant mouse gelatinase B (110 kDa) with N-glycanase resulted in conversion to a 105 kDa gelatinase B without loss of zymographic activity (data not shown). The 26 kDa fragment is glycosylated by the microsomal membrane preparation. This fragment represents the amino-terminus of the mouse gelatinase B since the N-glycosylation sites are all situated in the NH₂-terminal third of the protein. The natural mouse gelatinase B contains approximately 10 kDa of carbohydrates (43). Moreover, in accordance with the predicted protein sequence, this 26 kDa NH₂-terminal fragment is most intense on the autoradiogram, and it contains 4 or 5 methionines (positions 1, 61, 62, 74 and 136 in the amino acid sequence). The other two fragments of 45 and 38 kDa (after translation in reticulocyte lysate) each contain only one or two methionines (positions 419, 469 and 605) and by [³⁵S]methionine labelling experiments consequently appear less intense on the autoradiogram. It is known that prolonged incubation or repeated freeze/thawing of matrix metalloprotease can lead to autoactivation and autoproteolysis (47, 48).

The recombinant mouse gelatinase B has gelatinolytic activity, binds to gelatin-Sepharose and cross-reacts with a polyclonal antibody raised against human gelatinase B. The similarity between the human and mouse gelatinase B proteins is 82 % (43). Mouse gelatinase B cDNA has been cloned by three other groups besides ours (42, 44, 45), but several differences are apparent between the four known cDNA sequences and between certain of these cDNA sequences and the genomic sequence (43). These differences, situated in the carboxy-terminal part of the enzyme, more precisely in the type V collagen-like domain and in the hemopexin domain, could lead to non-conservative amino acid substitutions in the protein. Although the nature and importance of these discrepancies remains to be determined, the cDNA we have cloned and sequenced codes for the first murine recombinant functional gelatinase B enzyme. Our results demonstrate that recombinant murine gelatinase B can transiently be expressed in heterologous cells. In accordance with recent data on the human gelatinase B, which show that permanent expression of high amounts of the recombinant form in rat tumor cells is selected against (38), in our hands expression of gelatinase B has negative effects on cell growth and survival. Therefore, yeast expression is developed. The availability of recombinant mouse gelatinase B protein and of DNA probes for this murine matrix metalloprotease is crucial for the other claims and for in vivo testing in animal models.

### 2.4. Figure legends

Figure 5. Construction of expression plasmids containing the complete mouse gelatinase B coding sequence. For details see text. EX=exon; pBS=pBluescript; B=BamHI, E=EcoRI, N=NotI, T=TthIII1, XB=XbaI, XH=XhoI; S1,S2=sense primers SPEX1 and SPEX2; A1,A2=antisense primers APEX1 and APEX2.

Figure 6. In vitro transcription and translation of pBKR/MMGELB DNA.
(A) Linearized plasmid DNA from pBKR/MMGELB was transcribed in vitro and the RNA then translated in rabbit reticulocyte lysate in the absence (-) or presence (+) of canine microsomal membranes (CMM). Protease inhibitors (PI; PMSF and antipain) were added to the indicated translation reactions. Lane CO shows a control translation reaction in the absence of added RNA. MM= molecular mass standards used for SDS/PAGE (in kDa). (B) Purified recombinant murine gelatinase B (mGEL-B) from pBKR/MMGELB-transfected CHO cells was incubated with 2 µl of reticulocyte lysate (RL) for 4 h and aliquots subsequently subjected to zymographic analysis (left panel) or to Western blot analysis (right panel). The 110 kDa gelatinase B band is indicated by arrowheads.

Figure 7. Expression of mouse gelatinase B in CHO cells (pRcCMV-vector).
(A) The construct RcCMV/MMGEXP was made as described in the experimental procedures section. The genomic DNA/cDNA hybrid was cloned between the CMV-promoter and the BGH polyadenylation signal of the expression vector pRcCMV. This vector contains the neomycin resistance gene. (B) Zymography of conditioned media (5 µl per lane) from untreated CHO cells (lane 3), control-transfected CHO cells (lane 2) and RcCMV/MMGEXP-transfected CHO cells (lane 1). Molecular mass standards are indicated in kDa at the right hand side. Bands representing recombinant murine gelatinase B activity are indicated by arrowheads at the left hand side. Note that the cells were cultured in medium containing 10% fetal bovine serum.

Figure 8. Expression of mouse gelatinase B in CHO cells (pBK-RSV-vector).
(A) The construct pBKR/MMGELB was made as described in the experimental procedures section. The genomic DNA/cDNA hybrid was cloned between the RSV-promoter and the SV40 polyadenylation signal of the expression vector pBK-RSV. This vector contains the neomycin resistance gene. (B) Zymography of recombinant mouse gelatinase B (lane 1: 5 µl of supernatant from transfected pool; lane 2: purified enzyme) compared to natural mouse gelatinase B from LPS-stimulated WEHI-3 cells (lane 3: 1 µl of cell culture supernatant; lane 4: purified enzyme). The 110 kDa gelatinase B band is indicated by an arrowhead. (C) Silver-stained SDS/PAGE of conditioned media (1 µl per lane; 24 h incubation period) from untreated CHO cells (lane C), control-transfected CHO cells (lane 1) and pBKR/MMGELB-transfected CHO cells (lane 2). Lane 3 shows an aliquot of the affinity-purified recombinant mouse gelatinase B enzyme and lane W shows a Western blot analysis on supernatant from gelatinase B-transfected CHO cells. The 110 kDa mouse gelatinase B band is indicated by arrowheads.

### EXAMPLE 3

### D-penicillamine and tetracyclines and derivatives thereof as gelatinase B inhibitors

### The gelatinase inhibitory activity of tetracyclines and chemically modified tetracycline analogues.

### 3.1. Introduction

D-penicillamine was discovered as an inhibitor of gelatinase B by zymography analysis and microtiterplate analysis. When 20 tetracycline analogues were compared the inventors found that the inhibitory factor (IF) at 1.67 mg/ml varied from O to 27. Chlortetracycline (IF= 27) and epichlortetracycline (IF= 9) were the most potent gelatinase inhibitors. The inventors dissociated the gelatinase B inhibitory activity of tetracyclines from their antimicrobial activity. In view of the pathophysiological function of gelatinases, the definition of gelatinase inhibitors with known efficacy, safety and side-effects is crucial for the treatment of diseases such as cancer invasion and metastasis and multiple sclerosis.

### 3.2. Materials and Methods

### Reagents

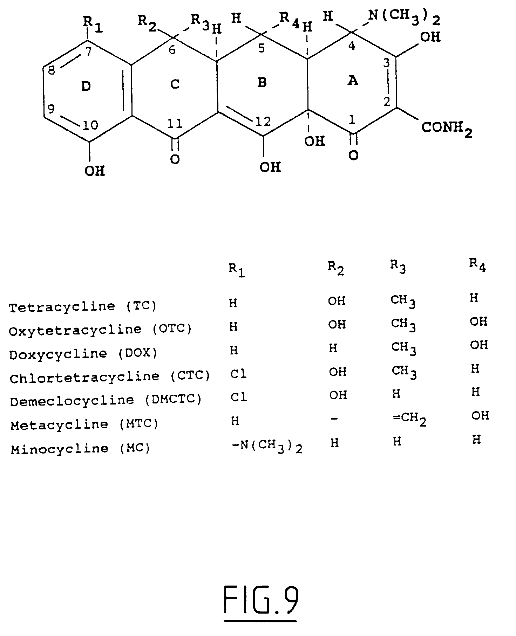

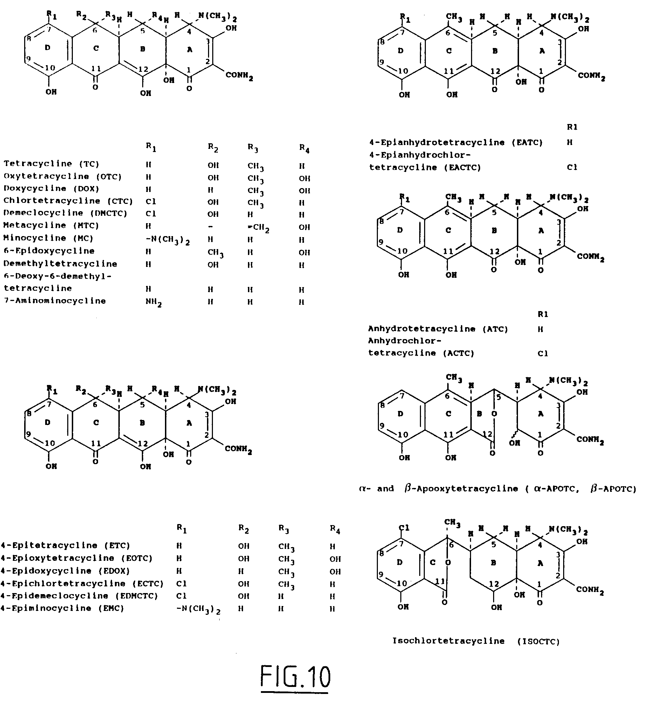

D-penicillamine was purchased from Gist-Brocades, The Netherlands. The tetracyclines and analogues mentioned in Table 1 are depicted in figures 9 and 10. Those indicated by an asterisk are commercially available from Acros Chimica, (Beerse, Belgium).
The test system used is described hereabove.

**Table 1**

| | tetracycline concentrations | | |
|---|---|---|---|
| Name | 3.33 mg/ml | 1.67 mg/ml | 0,83 mg/ml |
| tetracycline HCl* | 8 | 3 | - |
| 4-epitetracycline HCl* | ND | 3 | - |
| anhydrotetracycline HCl* | - | - | - |
| 4-epianhydrotetracycline HCl* | precipitation | 3 | 2 |
| oxytetracycline base* | precipitation | - | - |
| 4-epioxytetracyline base* | 20 | 3 | - |
| α-apooxytetracycline base* | 9 | 3 | - |
| β-apooxytetracycline base* | 3 | - | - |
| doxycycline hyclate | - | - | - |
| 6-epidoxycycline HCl | - | - | - |
| metacycline HCl | 27 | 8 | 3 |
| demethylchlortetracycline HCl | - | - | - |
| 4-epidemeclocycline HCl | - | - | - |
| demethyltetracycline base | - | - | - |
| chlortetracycline HCl* | 45 | 27 | 9 |
| 4-epichlortetracycline HCl* | precipitation | 9 | 3 |
| isochlortetracycline HCl* | 3 | - | - |
| anhydrochlortetracycline HCl* | 6 | 2 | - |
| 4-epianhydrochlortetracycline HCl* | 3 | - | - |
| minocycline HCl | 8 | 3 | - |
| 4-epiminocycline HCl | 3 | - | - |
| 7-aminominocycline HCl | precipitation | - | - |
| 6-deoxy-6-demethyltetracycline HCl | 8 | 5 | 3 |

### Titration of gelatinase B in the microtiter plate assay

On a theoretical basis the developed test is useful for both enzyme and enzyme-inhibitor assays. Previously, the inventors have shown that neutrophils do not produce the natural tissue inhibitor of metalloprotease (TIMP-1), or gelatinase A (8). In experiments a standard preparation, containing purified homogenous gelatinase B from human neutrophils yielded a titration curve parallel with those of crude preparations of neutrophil gelatinase B in the plate assay. For all further tetracycline inhibition experiments, the crude preparation of neutrophil gelatinase B was used.

The change in optical density with respect to the change in enzyme concentration is sigmoidal (Fig. 3). The titration curves of gelatinase inhibition by tetracyclines were always related to a blanc curve and this for enzyme concentrations within the slope of the curve (vertical dotted lines Fig. 3). The decrease in enzyme activity to which the tetracycline incubated sample corresponded was defined as the inhibition factor (IF).

With the classical zymography technique it was found that minocyclin inhibits gelatinase B in a dose-dependent way (Fig. 4). Therefore, 23 tetracyclines and CMTCs were tested for their inhibitory acitivity in the gelatinase microtiter plate assay (table 1). Three dilutions were tested, starting from 3.33 mg/ml. In some cases due to the amount of material available or due to the chemical properties of the tetracycline, a highest concentration of only 1.67 mg/ml was used. Several tetracyclines inhibited gelatinase B in a dose-dependent way. Chlortetracycline HCl was the most potent inhibitor, followed by 4-epichlortetracycline. The inhibitory activity of minocycline in the zymography assay was confirmed in the plate assay. Several tetracyclines and CMTCs did not show any inhibitory activity.

### Tetracyclines as inhibitors of gelatinase B and correlation with their antimicrobial potency

Fig. 9-10 illustrate the structure modifications of the tetracyclines and CMTCs with known antimicrobial potency as well as their anti-gelatinase activity.

It appears that the inhibitory activity is not linked to the antibiotic potency. Chlortetracycline is more active as a gelatinase inhibitor than the antibiotically more potent minocycline, while the potent antibiotic doxycycline is not active as an inhibitor. Oxytetracycline is less active as an matrix metalloprotease inhibitor than its 4-epimer 4-epioxytetracycline which is only weakly potent as antibiotic.

Chlortetracycline is much more active than tetracycline. But minocycline which carries a dimethylamino group at C7 is also active and demethylchlortetracycline which, compared to chlortetracycline, lacks a methyl group at C6 is not active. So it appears that the structure at C6 is important too. Metacycline carries a methylene at C6 and is very active, while doxycycline with a methyl group at C6 is not active, and minocycline, with no substituents at C6 is active. The configuration at C4 also plays a role. In several cases the 4-epimers are less active (4-epichlortetracycline, 4-epinanhydrochlortetracycline, 4-epiminocycline), in some cases they are more active (4-epianhydrotetracycline, 4-epioxytetracycline) and for tetracycline no effect is observed.

### 3.3. Summary

In view of the disease-promoting role of metalloproteases in chronic inflammations (26) and cancer cell invasion and metastasis (27, 28, 29), potent metalloprotease inhibitors might become important drugs for the treatment of these diseases. Therefore the inventors established a microtiter plate assay for matrix metalloproteases and inhibitors, as described hereabove, and compared the inhibitory effect of a series of tetracycline analogues and D-penicillamine.

The described assay is based on the digestion of biotinylated gelatin by gelatinases and quantification of residual biotinylated gelatin by peroxidase-streptavidin. With the use of laboratory standard preparations this microtiter plate assay can be employed to quantify gelatinases in crude supernatants and to test for inhibitors of gelatinases.

In contrast to the zymography inhibitor assays, the microtiter plate assay is less labour-intensive and can be performed with at least 1000-fold less inhibitor. To exemplify its usefulness and to determine the structure-function effects we studied the inhibitory effect of a series of tetracyclines and tetracycline analogues. Chlortetracycline was found to be the most active, followed by metacycline HCl and 4-epioxytetracycline base. The inventors compared the activities of the gelatinase-inhibiting tetracyclines and CMTCs with their known antimicrobial properties. It appears that the gelatinase inhibitory activity and antimicrobial potency of the molecules can be dissociated. This is in line with and extends earlier findings (30,31) on inhibition of neutrophil collagenase with tetracycline analogues. Based on the structure of the different CTs and CMTCs and their activity in the gelatinase microtiter plate assay it can be concluded that the substitution and the configuration of the structures on C6 and C7 influence the activity but apparently these influences are complex. From the available information it is impossible to predict which structure will be the most effective inhibitor. It is also impossible to discuss in detail the relation of the inhibitory activity of the tetracyclines to their chelating properties, since the relative chelating strength of all the tetracyclines examined is not available. It has been reported that the chelating properties of demethylchlortetracycline, doxycycline and minocycline towards Fe³+, Cu²+, Ni²+ and Co²+ are comparable (32) while it was found that their ability to block gelatinase activity is different. Experiments with chlortetracycline, tetracycline hydrochloride and metacycline also suggest that the gelatinase inhibitory activity of some of the tetracyclines is not completely attributable to their ability to chelate Ca²+ (data not shown).

The anti-metalloprotease activity of tetracyclines has been found to be effective mainly against collagenases of inflammatory (neutrophil) origin but not against fibroblast collagenase (32). This may prove a double advantage for their therapeutic use. It would provide a potent inhibitor of neutrophil damage, relatively resistant to other metalloprotease inhibitors such as α2-macroglobulin. Additionally, its specificity towards neutrophil collagenases decreases the risk of tetracycline therapy interfering with normal tissue remodelling and repair (30).

### 3.4. Legends to tables and figures

Table 1. Inhibition factor (IF) of tetracycline analogues as determined in the gelatinase microtiter plate assay. Where indicated, precipitation of the tetracycline at high concentrations did not allow an accurate determination of the IF. ND: not determined. Asterisks indicate commercially available tetracyclines.

Figure 9 and 10. Structures of the tetracyclines.

Figure 3. Determination of the inhibition factor. A standard preparation of gelatinase B from human neutrophils was spectrophotometrically titrated in the microtiter plate assay by digesting biotinylated gelatin. Addition of anhydrochlortetracycline (3.33 mg/ml to 0.09 mg/ml shifted the titration curves in a parallel way and enabled to determine the inhibitory factor (IF).

Figure 4. Inhibition of gelatinase B by minocycline on zymography. A standard preparation of gelatinase B from neutrophils was repetitiously loaded in the slots of a zymography gelatin substrate gel. After electrophoretic separation the gel was sliced every three lanes and the slices were incubated in test tubes in developing buffer without and with increasing amounts of gelatinase B inhibitor. The enzyme activity was developed by staining of the substrate with Coomassie blue and quantification of the substrate digestion by scanning densitometry. Percent inhibition is related to a control to which no minocycline was added. The 92 kDa neutrophil gelatinase is indicated with an arrowhead.

### EXAMPLE 4

### Antibodies and natural inhibitors and derivatives as gelatinase B inhibitors

### 4.1. Introduction

The inhibitory activity of antisera can not be tested using zymography because the antibodies need to diffuse into the substrate gel. The same holds true for other macromolecular inhibitors of gelatinases, for instance tissue-inhibitors of metalloproteases. The assay decribed here above, however, makes it possible to define macromolecular inhibitors of gelatinases.

Pure gelatinase B (7) was used to raise polyclonal antisera in rabbits and mice. The antisera were tested in the microtiterplate assay (as described hereabove) as inhibitor of gelatinase B. By hybridoma technology, mouse monoclonal antibodies against gelatinase B were generated. At least one of these given the name REGA 1, was found to inhibit the gelatinase B.

### 4.2. Materials and methods

The polyclonal rabbit antibodies (Ab1 and Ab2) raised against purified human Malavu cell gelatinase and neutrophil gelatinase respectively were obtained as described (8). The mouse antibodies (Ab3) were generated by repeated injection of purified neutrophil gelatinase (3) in Balb/c mice and blood sampling. Alkaline-phosphatase-conjugated anti-rabbit and anti-mouse antisera were purchased from DAKO/PROSAN, Glostrup, Denmark.

### Western blotting

Standard neutrophil gelatinase (1µl) was seperated on a 15% SDS/PAGE gel and transferred to a polyvinyldifluoridon (PVDF) membrane. The membranes were blocked in 3% bovine albumin serum in Tris-buffered saline (TBS) for one hour and then incubated overnight in a 1/5000 dilution of the primary antisera in TBS. After washing, the membranes were incubated with an alkaline-phosphatase-conjugated anti-rabbit or anti-mouse antibody (1/2000 dilution), depending on the origin of the primary antibody, and stained with nitroblue tetrazolium and bromochloroindolylsulphate until bands appeared.

### Inhibition of gelatinase B by specific antisera

The figures illustrate the reactivity of neutrophil gelatinase B activity with 3 different polyclonal antibodies (Ab1, Ab2 were generated in rabbit (7,8), Ab3 in mouse) directed against malavu cell gelatinase (Ab1) and purified human neutrophil gelatinase (Ab2, Ab3), respectively.

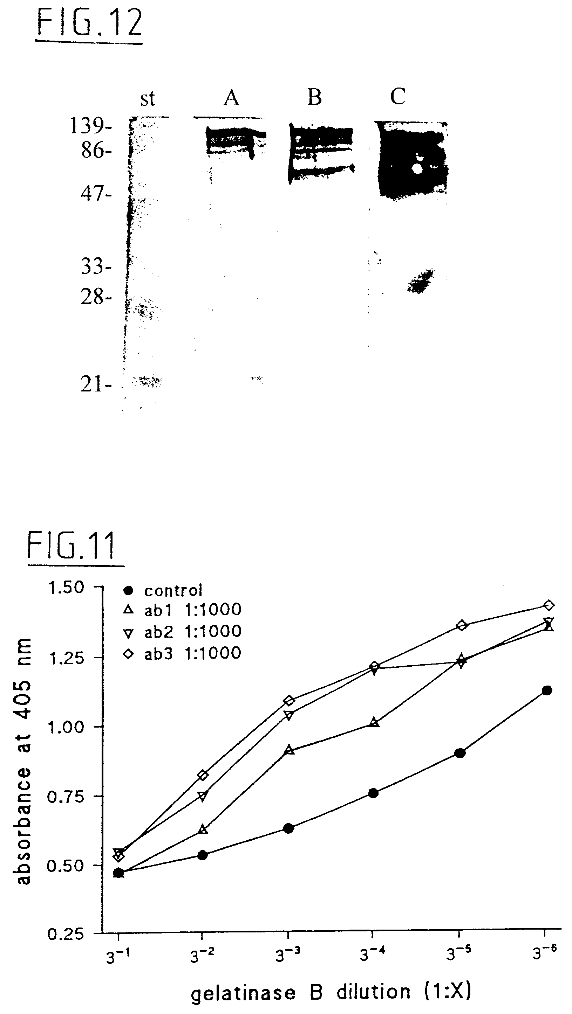

All three antibodies (dilution 1:1000) inhibit the activity of the standard gelatinase B solution (diluted 9 times or more) (Fig. 11). At a gelatinase B dilution of1/27 the inhibition factors were 9, 22, and 27 for Ab1, Ab2 and Ab3 respectively.

Figure 12 demonstrates the reactivity ofthe three antisera to the standard neutrophil gelatinase B preparation in a Western blot. Preimmune sera from rabbit and mouse did not react with gelatinase in the western blot and were not inhibitory at a dilution of 1:1000, whereas Ab1, Ab2 and Ab3 clearly reacted specifically with the antigen.

### 4.3. Figure legend

11) Inhibition of standard gelatinase B acitivity by specific antisera generated against human malavu-cell gelatinase (ab1) or human neutrophil gelatinase (ab2, ab3) in rabbit (ab1, ab2) or mouse (ab3).
12) Reactivity of the antisera ab1 (A), ab2 (B) and ab3 (C) with the standard gelatinase B preparation in a western blot. st, molecular weight standard.

### EXAMPLE 5

### Use of D-Pen alone or in combination with tetracyclines for the treatment of demyelinating (neurological) diseases

### 5.1. Introduction

Gelatinase B, a marker enzyme for demyelination in multiple sclerosis (MS), was dose-dependently inhibited by D-penicillamine. The inventors describe the discovery that at therapeutic doses of D-penicillamine, this inhibition was extended to complete blockage of hyperacute experimental allergic encephalomyelitis (EAE) in mice, an animal model of acute demyelination. In a chronic relapsing model of EAE in Biozzi mice, D-penicillamine abrogated the exacerbations.

Gelatinase B is further controlled by specific inhibitors, the tissue inhibitors of metalloproteases. The latter are transcriptionally regulated by cytokines such as TGF-β (13). The relative contribution of various cytokines, including chemokines, in the balance of the protease load and the extracellular processing of structural proteins such as myelin basic proteins have recently been proposed as the REGA-model (remnant epitope generates autoimmunity) to explain the biochemical basis and the early events in CNS demyelination (14). Cytokine-regulated extracellular proteolysis generates peptides from intact proteins which are at the origin of the autoimmune process.

The inventors demonstrate that inhibition of the extracellular protease-cascade is an effective and alternative strategy for MS treatment in addition to cytokine blockage or various T-cell depletion or anergizing methods such as T-cell vaccination (15), T-cell depletion by anti CD4 or anergy (16) or oral tolerance (17) or synthetic peptides or peptidomimetics (18).

### 5.2. Experiments

### In vitro inhibition of gelatinase B in the CSF of patients with multiple sclerosis and optic neuritis

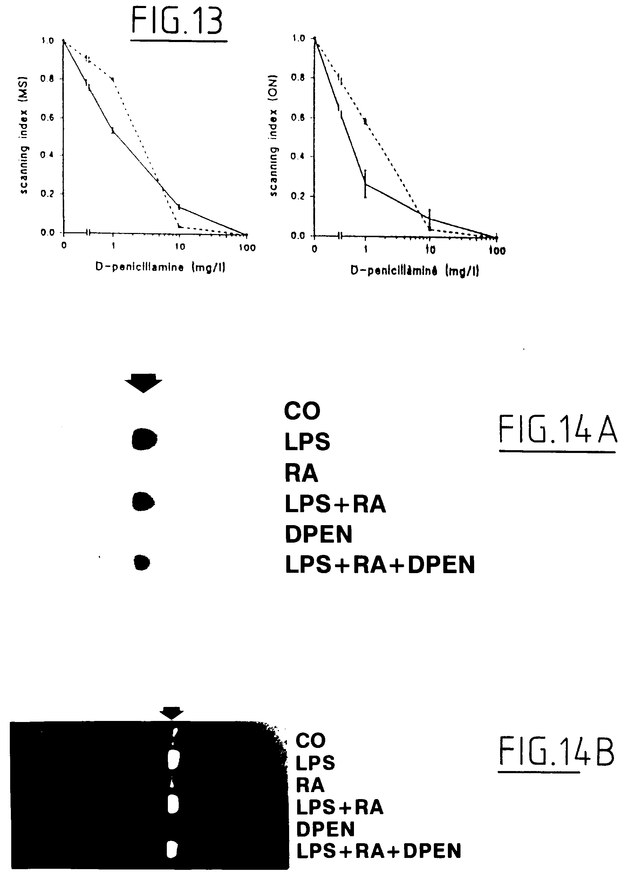

Gelatinase A and B, detected by zymography in the cerebrospinal fluid of MS patients was inhibited in a dose-dependent way with D-penicillamine (Fig. 13). Similar results were obtained when pure preparations of gelatinase B were tested or when gelatinase B was measured in the presence of D-penicillamine in body fluids or cell supernatants. Although these results suggested a direct effect by D-penicillamine on gelatinase activities, in order to understand the inhibitory effect it was verified whether D-penicillamine could interfere with gelatinase B production or indirectly affect the gelatinase activities in vivo.

### Discovery of the mechanism of action of D-penicillamine as gelatinase B inhibitor

The spontaneous and the induced levels of gelatinase B protein and mRNA remained constant (Fig. 14). Instead, it was found that D-penicillamine inhibited both urokinase (u-PA) and tissue-type plasminogen activator (t-PA), two enzymes involved in the protease cascade leading to gelatinase activation. D-penicillamine also had direct chelating effect on the catalytic zinc and the conformational calcium ions. The ID₅₀ for homogeneous u-PA, t-PA and gelatinase B were 14, 18 and 3mg/l, respectively.

### Discovery of the in vivo beneficial effects of D-penicillamine in demyelinating diseases

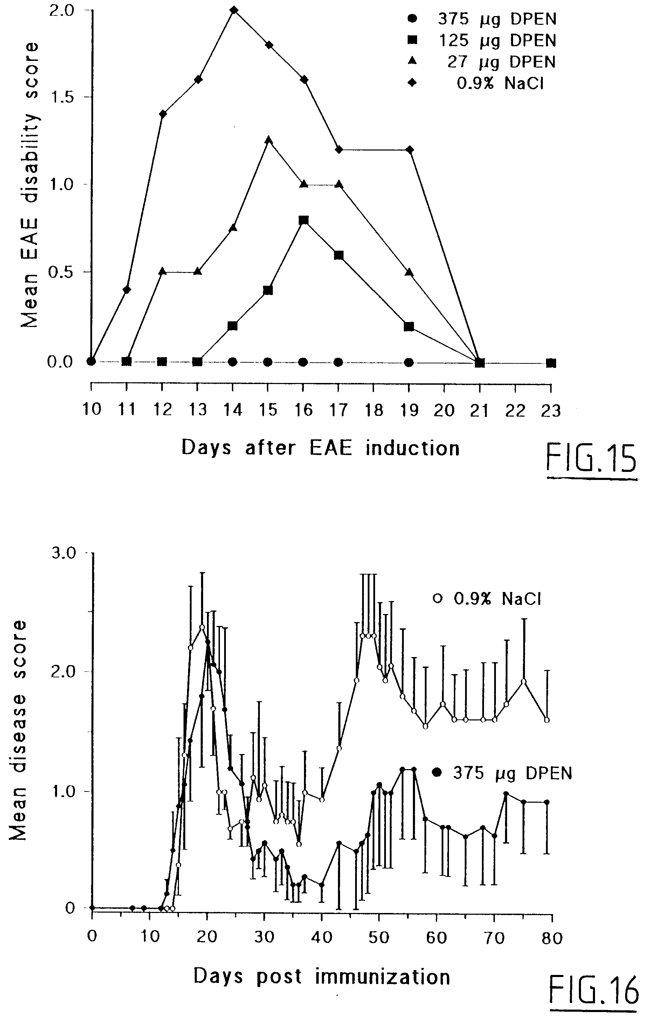

In view of these findings we tested the effects of D-penicillamine on the clinical outcome of EAE in mice (Acute EAE was induced in groups of 5-10 SJL mice (7.5weeks old) by injection of 0.75mg spinal cord homogenate from NMRI mice in 50µl PBS to which 0.2mg M.tuberculosis was added in 50µl complete Freunds adjuvans. In both hind paws 50µl of this emulsion was injected on day 0. On day 0 and 2, 50µl B.pertussis (200x10⁹ bacteria/ml) were injected intraveneously. Treatment was done by injection on day 0, 2, 5, 7, 9, 12, 14, 16 and 19 of 100µl D-penicillamine solution intraperitoneally. The control group received injections with 0.9% NaCl, used as excipiens. The animals were daily monitored and the disease and mortality were scored.). Figure 15 shows that D-penicillamine, when administered during the onset of symptoms, inhibits in a dose-dependent way the development of disease symptoms in mice developping EAE. When given the time-point when the mice started to develop clinical symptoms the treatment proved still to be effective. In a model of chronic relapsing EAE in Biozzi AB/H mice (Chronic EAE was developped in Biozzi mice by injection of myelin. The animals in this group were only treated after they all developped disease. The scores were daily monitored.), D-penicillamine abrogated the disease recurrencies (fig. 16) even when administered after the animals had developed full-blown disease. This is of particular interest in the treatment of the recurrencies in MS, in which disease so far no efficaceous treatment of the autoimmune process has been found.

### 5.3. Summary

Protease antagonization has since long been proposed as a treament strategy for demyelinating diseases, including MS (34), but the proteases involved were not well characterized. Based on the inventors' earlier search of such proteases and in particular the studies with gelatinase B, the inventors show that targetted protease inhibition with available peroral drugs limits the demyelinating disease. The inventors show that the treatment with D-penicillamine not exclusively results in gelatinase B-inhibition. D-penicillamine also inhibits the plasminogen activators urokinase and tissue-type plasminogen activator which act in the protease cascade leading to gelatinase B activation. It may also interfere with membrane-associated metalloproteases involved in cytokine activation or myelin degradation (35). We here provide data which incite clinical trials of gelatinase B-inhibition for the treatment of MS and other demyelinating diseases and advocate that in double blind clinical trials of novel MS-treatments the placebo control be replaced by e.g. D-penicillamine, a drug with known effects and side-effects in humans.

Previous studies of the plasminogen (activator) inhibitors in MS had mixed results depending on the clinical status (34). Gelatinase B was not observed in all patients with MS or other inflammatory neurological diseases, but it can easily be detected by SDS zymography. Therefore, in clinical trials with D-penicillamine, a directed evaluation is now possible depending on the determination of the CSF gelatinase B status.

The mechanism of action of D-penicillamine has been deciphered and is multiple. First, it directly inhibits gelatinase B possibly by chelating the conformational calcium ions or the Zn atom of this metallo-enzyme. Second, at the ID₅₀ for gelatinase B it also inhibits t-PA and u-PA and thus indirectly prevents the activation of progelatinase B to gelatinase B. Third, metallo-enzyme chelating inhibitors might block the TNF-α processing protease and thus block TNF-α action in transcriptionally activating the gelatinase B gene (36). Because TNF-α seems to be a disease promoting cytokine in MS and EAE (14) inhibition of this cytokine belongs to the MS treatment strategies.

EAE as - model for MS has to be used with caution (14). The cells involved in these pathologies and the clinical outcome differ considerably. Indeed, in contrast to MS, EAE is most often a monophasic disease with a predictable outcome. In view of the fact that the relapse in MS is unpredictable this implies that a chronic treatment (with monitoring of side effects) with D-penicillamine would have to be instated to block the MS relapses. Our results with the relapsing model in Biozzi mice are particularly important, because we provide evidence that the symptoms of demyelination can be curtailed and the disease can be reduced by starting the treatment only after the disease has clinically developed. Such evidence is most relevant in an analogous treatment of MS.

Finally, gelatinase B and other metalloproteases can efficiently be inhibited by TIMPs and this might be one of the rationales behind the effect of TGF-β in animal models for MS. TIMP and cytokine therapies for MS (e.g. IFN-β) or cytokine antagonization with natural or engineered proteins have, however, the intrinsic disadvantage of the need for parenteral administration.

In conclusion, the inventors provide evidence that D-penicillamine or derivatives and combinations with other gelatinase B inhibitors (e.g. tetracyclines and derivatives, inhibitory polyclonal and monoclonal antibodies and derivatives thereof) are effective in MS treatment and disclose their mode of action. Because some of these are clinically familiar drugs, they are a MS therapeutics and attractive references to be compared with the expensive molecular drugs under current investigation.

### 5.4. Legend to Figures

Figure 13. Inhibition of gelatinases in the CSF of patients with multiple sclerosis or optic neuritis. Gelatinase A (---) and B(_) activities in the CSF were measured by zymography in the absence or the presence of increasing concentrations of D-penicillamine. The scanning indices for each patient are calculated at the residual activity in the presence of inhibitor versus the activity without inhibitor. The scanning index is expressed as the mean (+/-) SEM, n=5) for patients with definitive MS and ON.

Figure 14. D-penicillamine does not inhibit cellular gelatinase B and mRNA production. Cultures of the human monocytic cell line THP-1 were stimulated with lipopolysaccharide (LPS, 1µg/ml), retinoic acid (RA, 100nM) or left untreated (CO) in the presence or absence of D-penicillamine (DPEN, 50 µg/ml). After 16 h total RNA was extracted and gelatinase B mRNA was measured by northern blot analysis with a human probe (41) (a). Gelatinase B protein in the cell culture fluids was assayed by zymographic analysis on gelatin substrate gels (b).

Figure 15. D-penicillamine inhibits acute EAE in mice.
Groups of 5 SJL mice were injected with spinal cord homogenate to induce hyperacute EAE. The mean disability index of 5 mice was scored versus days after immunization. Control mice received 0.9% NaCl i.p. and were compared with groups which were treated with the equivalent of 27 to 375 µg D-penicillamine per mouse daily.

Figure 16. D-penicillamine inhibits chronic EAE in mice.
Groups of 8 Biozzi AB/H mice were injected with spinal cord homogenate to induce chronic relapsing EAE. After the first episode of encephalitis (from day 22 onwards) the treatment with D-penicillamine was initiated at 750 µg, three times weekly, and the mean clinical score (average of disease grade for each group of mice +/- standard error) was expressed versus time post immunization.

### EXAMPLE 6

### Use of gelatinase inhibitors (e.g. D-penicillamine alone or in combination with tetracyclines) for the treatment of invasive cancers.

The gelatinases and plasminogen activators u-PA and t-PA have been implicated in invasion and metastasis of cancer cells. The inventors have discovered that the single agent, D-penicillamine, can inhibit all these enzymes. This is achieved at the therapeutical dosis currently used in the chronic treatment of rheumatoid arthritis.

In addition and because gelatinase B is the terminal enzyme in the cascade leading to matrix degradation (comprising plasminogen activators, plasminogen, collagenase, stromelysin and gelatinases), inhibition of gelatinase by more specific inhibitors (e.g. tissue inhibitors and the herementioned polyclonal and monoclonal antibodies) is a use of the reagent (REG 1) and derivatives thereof in treatment of invasive cancers.

### Cited references

1. Opdenakker, G., Masure, S., Grillet, B. & Van Damme, J. (1991) Cytokine-mediated regulation of human leukocyte gelatinases and role in arthritis. Lymphokine Cytokine Res. 10, 317-324
2. Hirose, T., Reife, R.A., Smith, G.N., Stevens, R.M., Mainardi, C.L. & Hasty, K.A. (1992) Characterization of type V collagenase (gelatinase) in synovial fluid of patients with inflammatory arthritis. J. Rheumatol. 19, 593-9
3. Kinane, D.F. (1992) Metalloproteinases in the pathogenesis of periodontal disease. Curr. Opin. Dent. 2, 25-32
4. Gijbels, K.S. Masure, S., Carton H. & Opdenakker G. (1992) Gelatinase in the cerebrospinal fluid of patients with multiple sclerosis and other inflammatory neurological disorders. Journal of Neuroimmunology, **41**, 29-34
5. Paemen, L., Olsson, T., Söderstrom, M., Van Damme, J. & Opdenakker, G. (1994) Evaluation of gelatinases and IL-6 in the cerebrospinal fluid of patients with optic neuritis, multiple sclerosis and other inflammatory neurological diseases. Eur. J. Neurol. 1,55-63
6. Woesner, F.J. (1991) Matrix metalloproteinases and their inhibitors in connective tissue remodeling. FASEB Journal 5, 2145-2154
7. Masure, S., Billiau, F., Van Damme, J. & Opdenakker G. (1990) Human hepatoma cells produce an 85 kDa gelatinase regulated by phorbol 12-myristate 13-acetate. Biochem. Biophys. Acta 1054, 317-325
8. Masure, S., Proost, P., Van Damme, J. & Opdenakker, G. (1991) Purification of 91-kDa neutrophil gelatinase. Release by the activating peptide interleukin-8. Eur. J. Biochem. 198, 391-398
9. L. Paemen, T. Olsson, M. Söderström, J. Van Damme, G. Opdenakker, Eur. J. Neurol. **1**, 55-63 (1994)
10. K. Gijbels et al., J. Neurosci. Res. **36**, 432 (1993); P. Proost, J. Van Damme, G. Opdenakker, Biochem. Biophys. Res. Commun. **192**, 1175 (1993)
11. S. Masure, P. Proost, J. Van Damme, G. Opdenakker, Eur. J. Biochem. **198**, 391 (1991); P. Proost et al., J. Immunol. **150**, 1000 (1992); P. Proost et al., Biochemistry, **32**, 10170 (1993)
12. G. Opdenakker, S. Masure, P. Proost, A. Billiau, J. Van Damme, FEBS Letters **284**, 73 (1991); J. Van Damme, P. Proost, J.-P. Lenaerts, G. Opdenakker, J. Exp. Med. **176**, 59 (1992); A.M.P. Montgomery, H. Sabsevari, R.A. Reisfeld, Biochem. Biophys. Acta, **1176**, 265 (1993)
13. D.R. Edwards et al., EMBO J. **6**, 1899 (1987)
14. G. Opdenakker, J. Van Damme, Immunol. Today **15**, 103-107 (1994)
15. J. Zhang, R. Medaer, P. Stinissen, D. Hofler, J. Raus, Science, **261**, 1451 (1993)
16. M.K. Waldor et al., Science **227**, 415 (1985); J.W. Lindsey et al., Neurology **44**, 413 (1994); C.C. Whitacre, I.E. Gienapp, C.G. Orosz, D. Bitar, J. Immunol. **147**, 2155 (1991)
17. H.L. Weiner et al., Science **259**, 1321 (1988); M.H.M. Wauben et al., Eur. J. Immunol. **24**, 1053 (1994); B.A. Jameson, J.M. McDonnell, J.C. Marini, R. Korngold, Nature, **368**, 744 (1994)
18. L. Adorini et al., Nature **334**, 623 (1988); M.H.M. Wauben et al., Eur. J. Immunol. **24**, 1053 (1994); B.A. Jameson, J.M. McDonnell, J.C. Marini, R. Korngold, Nature **368**, 744 (1994)
19. Arsenis, C., Moak, S.A. & Greenwald, R.A. (1992) Tetracyclines TETs) inhibit the synthesis and/or activity of cartilage proteinases in vivo and in vitro. Matrix-Suppl. 1, 314
20. Golub, L.M., Greenwald, R., Ramamurthy, N., Zucker, S., Ramsammy, L. & McNamara, T. (1992) Tetracyclines (TCs) inhibit matrix metalloproteinases (MMPs): in vivo effects in arthritic and diabetic rats and new in vitro studies. Matrix-Suppl. 1, 315-316
21. Vallee, B. & Auld, D. (1990) Zinc coordination, function, and structure of zinc enzymes and other proteins. Biochem. 29, 5647-5659
22. Gabler, W.L. & Creamer, H.R. (1991) Suppression of human neutrophil functions by tetracyclines. J. Periodontal Res. 26, 52-58
23. Golub, L.M., Suomalainen, K. & Sorsa, T. (1992) Host modulation with tetracyclines and their chemically modified analogs. Curr. Opin. Dent. 2, 80-90
24. Rifkin, B.R., Vernillo, A.T. & Golub, L.M. (1994). Blocking periodontal disease progression by inhibiting tissue destructive enzymes: a potential therapeutic role for tetracyclines and their chemically modified analogs. J. Periodontol. 64, 819-827
25. Sorbi, D., Fadly, M., Hicks, R., Alexander, S. & Arbeit, L. (1993) Captopril inhibits the 72 kDa and 92 kDa matrix metalloproteinases. Kidney Intern. 44, 1266-1272
26. Opdenakker, G. & Van Damme, J. (1994) Cytokine-regulated proteinases in autoimmune diseases. Immunol. Today 15, 103-107
27. Opdenakker, G. & Van Damme, J. (1992) Cytokines and proteinases in invasive processes: molecular similarities between inflammation and cancer. Cytokine 4, 251-258
28. Vassali, J.D. & Pepper, M. (1994) Membrane proteinases in focus. Nature 370, 14-15
29. Sato, H., Takino, T., Okada, Y., Cao, J., Shinagawa, A., Yamamoto, E. & Seiki, M. (1994) A matrix metalloproteinase expressed on the surface of invasive tumour cells. Nature 370, 61-64
30. Golub, L.M., Ramamurthy, N.S., McNamara, T.F., Greenwald R.A. & Rifkin, B.R. (1991) Tetracyclines inhibit connective tissue breakdown: new therapeutic implications for an old family of drugs. Crit. Rev. Oral Biol. Med. 2, 297-321
31. Golub, L., Lee, M., Lehrer, H., Nemiroff, G., McNamara, A., Kaplan R. & Ramamurthy, N. (1983). Minocycline reduces gingival collagenolytic activity during diabetes: preliminary observations and a proposed new mechanism of action. J. Periodont. Res. 18, 516
32. Suomalainen, K., Halinen, S., Ingman, T., Lindy, O., Saari, H., Konttinen, Y., Golub, L. & Sorsa, T. (1992) Tetracycline inhibition identifies the cellular sources of collagenase in gingival crevicular fluid in different forms of periodontal diseases. Drugs Exp. Clin. Res. 18, 99-104
33. G. Opdenakker, J. Van Damme, Cytokine 4, 251 (1992); K. Norga et al., unpublished results.
34. U.K. Rinne, P. Riekkinen, Acta. Neurol. Scand. **44**, 156 (1968); M.L. Cuzner, A.N. Davison, P. Rudge, Ann. Neurol. **14**, 337 (1978); C.F. Brosnan, W. Cammer, W.T. Norton, B.R. Bloom, Nature **285**, 235 (1980)
35. A. Chantry, P. Glynn, Biochem. J., **268**, 245 (1990); G.M. Mc. Geehan et al., Nature **370**, 558 (1994); A.R. Gearing et al., Nature **370**, 555 (1994); K.M. Mohler et al., Nature **370**, 218 (1994)
36. V. Lefebre, C. Peeters-Joris, G. Vaes, Biochim. Biophys. Acta, **1094**, 8 (1991); C.A. Patridge, J.J. Jeffrey, A.B. Malik, Am. J. Physiol. **265**, L438 (1993); C. Ries, H. Kolb, P.E. Petrides, Blood **83**, 3638 (1994)
37. S. Masure, G. Nys, P. Fiten, J. van Damme, G. Opdenakker, Mouse gelatinase B: cDNA cloning, regulation of expression and glycosylation in WEHI-3 macrophages and gene organisation. Eur. J. Biochem. 218, 219-141 (1993)
38. Bernard, E.J., Gruber, S.B. & Muschel, R.J. (1994) Direct evidence linking expression of matrix metalloproteinase 9 (92-kDa gelatinase/collagenase) to the metastatic phenotype in transformed rat embryo cells, Proc. Natl. Acad. Sci. USA 91, 4293 - 4297
39. Murphy, G. & Docherty, A.J.P. (1992) The matrix metalloproteinases and their inhibitors, Am. J. Respir. Cell Mol. Biol. 7, 120 - 125
40. Collier, I.E., Wilhelm, S.M., Eisen, A.Z., Marmer, B.L., Grant, G.A., Seltzer, J.L., Kronberger, A., He, C., Bauer, E.A. & Goldberg, G.I. (1988) H-ras oncogene-transformed human bronchial epithelial cells (TBE-1) secrete a single metalloprotease capable of degrading basement membrane collagen, J. Biol. Chem. 263, 6579 - 6587
41. Wilhelm, S.M., Collier, I.E., Marmer, B.L., Eisen, A.Z., Grant, G.A. & Goldberg, G.I. (1989) SV40-transformed human lung fibroblasts secrete a 92-kDa type IV collagenase which is identical to that secreted by normal human macrophages, J. Biol. Chem. 264, 17213 - 17221
42. Tanaka, H., Hojo, K., Yoshida, H., Yoshioka, T. & Sugita, K. (1993) Molecular cloning and expression of the mouse 105-kDa gelatinase cDNA, Biochem. Biophys. Res. Commun. 190, 732 - 740
43. Masure, S., Nys, G., Fiten, P., Van Damme, J. & Opdenakker, G. (1993) Mouse gelatinase B. cDNA cloning, regulation of expression and glycosylation in WEHI-3 macrophages and gene organisation, Eur. J. Biochem. 218, 129 - 141
44. Graubert, T., Johnston, J. & Berliner, N. (1993) Cloning and expression of the cDNA encoding mouse neutrophil gelatinase: demonstration of coordinate secondary granule protein gene expression during terminal neutrophil maturation, Blood 82, 3192 - 3197
45. Reponen, P., Sahlberg, C., Munaut, C., Thesleff, I. & Tryggvason, K. (1994) High expression of 92-kD type IV collagenase (gelatinase B) in the osteoclast lineage during mouse development. J. Cell Biol. 124, 1091 - 1102
46. Chu, G., Hayakawa, H. & Berg, P. (1987) Electroporation for the efficient transfection of mammalian cells with DNA, Nucleic Acids Res. 15, 1311 - 1326
47. Knäuper, V., Osthues, A., DeClerck, Y.A., Langley, K.E., Bläser, J. & Tschesche, H. (1993) Fragmentation of human polymorphonuclear-leucocyte collagenase, Biochem. J. 291, 847 - 854.
48. Crabbe, T., Ioannou, C. & Docherty, A.J.P. (1993) Human progelatinase A can be activated by autolysis at a rate that is concentration-dependent and enhanced by heparin bound to the C-terminal domain, Eur. J. Biochem. 218, 431 - 438

## Claims

1. Protease inhibitors for use in treating tissue destructive diseases such as arthritis, multiple sclerosis, and optic neuritis.

2. Protease inhibitors for use in treating cancer.

3. Protease inhibitors according to claims 1 or 2, wherein the protease inhibitors are metalloprotease inhibitors.

4. Protease inhibitors according to claim 3, wherein the metalloprotease inhibitors comprise tetracycline derivatives.

5. Protease inhibitors according to claim 4 wherein the tetracycline derivatives are selected from the group consisting essentially of: metacycline HCl, minocycline HCl, 4-epiminocycline HCl, 6-deoxy-6-demethyltetracycline HCl.

6. Protease inhibitors according to claim 3, wherein the metalloprotease inhibitors are macromolecular compounds such as sera, antibodies, fragments and modified versions thereof which are directed against proteases.

7. Protease inhibitors according to any one of the preceding claims, **characterized in that** the inhibitors are monoclonal antibodies or fragments thereof, directed against human gelatinase B from neutrophilic cells, obtainable by immunization of mice with human gelatinase B from neutrophilic cells, fusion of their spleen cells with a myeloma cell line, expansion of the resulting culture and selection of individual clones.

8. Protease inhibitors according to claim 7, selected from the group consisting essentially of the antibodies ab1, ab2 and ab3.

9. Protease inhibitors according to any previous claim, wherein the proteases to be inhibited belong to the group consisting essentially of gelatinases, plasminogen activators and processing proteases.

10. Protease inhibitors according to claim 9, wherein the gelatinase to be inhibited is gelatinase B.

11. Protease inhibitors according to claim 9, wherein the plasminogen activator to be inhibited is urokinase and/or tissue plasminogen activator.

12. Protease inhibitor according to claim 9, wherein the processing proteases to be inhibited comprise TNF-α processing protease.

13. Medicament for treating tissue destructive diseases such as multiple sclerosis, arthritis and optic neuritis comprising at least one of the members selected from the group consisting essentially of D-penicillamine, tetracycline and at least one of the protease inhibitors as claimed in claims 3 - 8.

14. Medicament for treating cancer based on at least one of the members selected from the group consisting essentially of D-penicillamine, tetracycline and at least one of the protease inhibitors as claimed in claims 3 - 8.

15. Tetracycline derivatives selected from the group consisting essentially of: metacycline HCl, minocycline HCl, 4-epiminocycline HCl, 6-deoxy-6-demethyltetracycline HCl.

16. Tetracycline derivatives according to claim 15 for use in inhibiting proteases according to claims 9 - 12.

17. Polyclonal and monoclonal antibodies and derivatives selected from the group consisting essentially of ab1, ab2, ab3 and REGA 1 for use in treating tissue destructive diseases such as multiple sclerosis, arthritis and optic neuritis.

18. Polyclonal and monoclonal antibodies and derivatives selected from the group consisting essentially of ab1, ab2, ab3, REGA 1 and others for use in treating cancer.

19. Use of at least one of the members selected from the group consisting essentially of D-penicillamine and tetracycline for the preparation of a medicament for treating tissue destructive diseases such as multiple sclerosis, arthritis and optic neuritis.

20. Use of at least one of the members selected from the group consisting essentially of tetracycline HCl, 4-epitetracycline HCl, anhydrotetracycline HCl, 4-epianhydrotetracycline HCl, oxytetracycline base, 4-epioxytetracyline base, α-apooxytetracycline base, β-apooxytetracycline base, chlortetracycline HCl, 4-epichlortetracycline HCl, isochlortetracycline HCl, anhydrochlortetracycline HCl, 4-epianhydrochlortetracycline HCl, D-penicillamine and tetracycline for the preparation of a medicament for treating cancer.

21. Use of anyone of chlortetracycline, metacycline HCl and 4-epioxytetracycline base, for the preparation of a medicament for treating tissue destructive disease or cancer.

22. Use of D-penicillamine as a protease inhibitor.

23. Use of tetracycline as a protease inhibitor.

24. Use of the antibodies ab1, ab2, ab3 and the inhibitory monoclonal antibody REGA 1 as protease inhibitors.

25. Use of at least two of the protease inhibitors according to claims 22 - 24 in combination as protease inhibitors.

26. DNA Construct for the expression of full-size mouse recombinant gelatinase B comprising a transcription initiation region, at least a part of the DNA-sequence according to figure A and a termination region aligned in the proper reading frame.

27. Process for measuring proteases and/or protease inhibitors comprising the following steps:
a) digestion of at least one predetermined substrate by the protease followed by
b) quantification of the residual non digested substrate.
